# EUROPEAN PATENT SPECIFICATION

(11) **EP 3 771 441 B1**
(45) Date of publication and mention of the grant of the patent: **06.04.2022**
(21) Application number: 19305991.2
(22) Date of filing: 31.07.2019
(51) Int. Cl.: A61B 17/16, A61B 17/92

(54) **HANDLE FOR MODULAR TOOL**
HANDGRIFF FÜR EIN MODULARES WERKZEUG
POIGNÉE POUR OUTIL MODULAIRE

(43) Date of publication of application: 03.02.2021
(73) Proprietor: Ostium Group, 44360 Saint Étienne De Montluc (FR)
(72) Inventor: BIBARD, Léopold, 44360 SAINT ETIENNE DE MONTLUC (FR); SOQUENNE, Edgard, 44360 SAINT ETIENNE DE MONTLUC (FR); RETAILLEAU, Vincent, 44360 SAINT ETIENNE DE MONTLUC (FR)
(74) Representative: Marks & Clerk France

(56) References cited:
- DE-U1- 9 217 486
- DE-U1-202012 104 364
- US-A- 5 409 492
- US-A- 5 788 701

## Description

### Field of the invention

The present invention relates to modular tools, and in particular a handle element for such tools.

### Background of the invention

The concept of modularity in tools is almost as old as tool use itself. Many ancient tool designs such as hammers, axes, picks and the like comprise a working element fixed to a handle by some means which allows the periodic replacement of one element or the other. Developments in manufacturing processes over the last two hundred years meanwhile have made it possible to develop releasable fixing mechanisms. For example, screw driver heads are commonly available as 6.35mm (quarter inch) hexagon bits, which may be inserted as required in a standard handle. The bits may be secured magnetically, or by means of a spring loaded ball bearing engaging a groove in the bit.In some contexts meanwhile, the operational constraints extant in that context may tend to lead away from such approaches. In the field of surgical instruments for example, the high forces involved along with sterilisation requirements may tend to indicate an all-in-one approach.

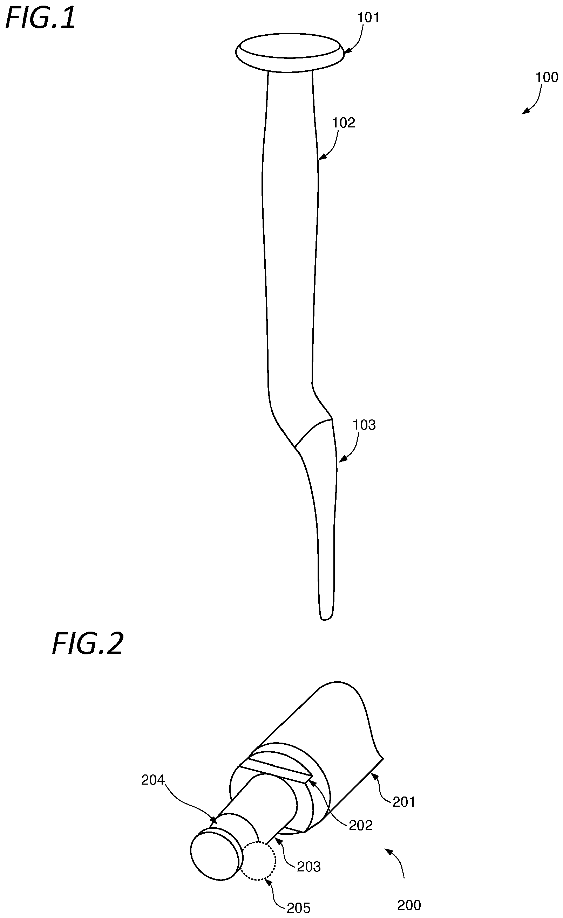

Figure 1 shows an orthopaedic reamer as known in the state of the art.

As shown in figure 1, a reamer 100 comprises a handle 102 with a strike head 101 and a reamer working surface 103. The reamer 100 is typically made entirely of stainless steel.

Notwithstanding the foregoing, the cost of manufacturing a complete set of standalone tools for example as shown in figure 1 can be considerable, and even in the field of surgical tools, some attempts at modular tools are known.

In this, as in any field where a solid positive engagement between the handle and further elements is a critical requirement, special consideration must be given to the securing mechanism.

Figure 2 shows a releasable securing mechanism for modular tools as known in the state of the art.

Figure 2 shows a conventional securing mechanism known in the art as a "Hudson Fitting". In particular, figure 2 shows a male Hudson fitting 200, attached to a tool element 201. The fitting comprises a cylindrical member 203 with a semi-circular channel or groove 204. When the cylindrical member 203 is slid into the corresponding female element, a spring loaded ball bearing 205 engages the channel so as to prevent accidental decoupling. In implementations where uncoupling must be avoided in the presence of a separating force, the ball bearing may be replaced with a removable cotter pin or the like. As shown, the fitting also comprises a flattened flange 202 at the proximal end of the fitting closest to the tool element 201. The flats of this flange may engage corresponding surfaces on the corresponding female element when the coupling is fully inserted, so that rotational forces may be effectively transferred between the two elements of the coupling.

DE9217486U1 describes a tow part orthopaedic reamer in which the two parts may be fitted together to define a cylindrical body, and where a strike head part is enclosed by a cylindrical sleeve which may be slide downward so as to enclose the point where the two parts are fitted together, so as to prevent their separation. The sleeve is provided with an internal detent which engages a spring loaded ball bearing when in the downward position.

Fittings such as that shown in figure 1 have been found unsatisfactory in terms of their ability to provide a solid positive engagement between the handle and further elements, whilst supporting uncoupling with a minimum of force and dexterity when required, yet averting the risk of accidental uncoupling. As such, it is desired to provide an improved coupling addressing some or all of these concerns.

### Summary of the invention

In accordance with the present invention in a first aspect there is provided a handle for a modular tool comprising a body having a distal end and a proximal end. The distal end provides a releasable coupling for an element of the modular tool, the releasable coupling comprising a cylindrical member provided with a first external helical thread, and a lateral slot. The slot opens on one periphery of the distal end of the body andwidens from the distal end towards the proximal end of the handle. The releasable coupling further comprises a threaded ring, the threads of the ring engaging the first external helical thread of the cylindrical member, the threaded ring being rotatable about the distal end of the member between an extended position in which the ring obstructs the slot opening on one periphery of the distal end of the body, and a retracted position in which the ring leaves the slot opening on one periphery of the distal end of the body unobstructed.

In accordance with a development of the first aspect, the lateral slot defines a T slot or dovetail.

In accordance with a further development of the first aspect, the dimensions of the lateral slot vary radially so that the force required to slide a corresponding keyed element of thesecondary element into the lateral slot increases as the corresponding keyed element of the tool progresses into the slot.

In accordance with a further development of the first aspect, the angular displacement of the threaded ring when rotated between the extended position and the retracted position is between 170 and 190 degrees.

In accordance with a further development of the first aspect, the threaded ring comprises an indentation at the distal edge thereof, the indentation being positioned on the circumference of the ring such that when the ring is in the retracted position the indentation is aligned with the slot.

In accordance with a further development of the first aspect, the cylindrical member is provided with a second external helical thread parallel the first external helical thread, and wherein the threads of the ring engage both the first external helical thread and the second external helical thread of the cylindrical member.

In accordance with a further development of the first aspect, the first helical thread has a first root to crest ratio, and the second helical thread has a second root to crest ratio, wherein the threads of the ring are defined so that the ring can only engage the corresponding respective helical thread, and thereby may only be mounted on the handle in one configuration.

In accordance with a further development of the first aspect, the pitch, the minor diameter, the major diameter, or the root to crest ratio of the of the first external helical thread varies along the length of the first external helical thread so that the force required to rotate the ring increases as the ring is rotated about the distal end of the member from the retracted position to the extended position.

In accordance with a further development of the first aspect, modular tool comprises a surgical instrument.

In accordance with a further development of the first aspect, modular tool is for surgery of the orthopaedic surgery or bonetraumatology.

In accordance with a further development of the first aspect, modular tool comprises a rasp or reamer or impactor.

In accordance with a further development of the first aspect, themodular tool comprises the handle, an interface part being releasably coupled to the handle and a working part being releasably coupled to the interface part.

In accordance with a further development of the first aspect, the handle is composed of a synthetic material or a synthetic composite material.

In accordance with a further development of the first aspect, the handle is composed of a glass fibre reinforced polyarylamide.

### Brief Description of the Drawings

The above and other advantages of the present invention will now be described with reference to the accompanying drawings, for illustration purposes only, in which:
- Figure 1: shows an orthopaedic reamer as known in the state of the art;
- Figure 2: shows a releasable securing mechanism for modular tools as known in the state of the art;
- Figure 3: shows a handle for a modular tool in accordance with an embodiment;
- Figure 4a: shows the handle in a first, retracted position prior to the insertion of the secondary element 305;
- Figure 4b: shows the handle in a first, retracted position after insertion of the secondary element 305;
- Figure 4c: shows the handle in a second, extended position after insertion of the secondary element 305;
- Figure 5a: shows a sectional view of the slot in a first embodiment;
- Figure 5b: shows a sectional view of the slot in a second embodiment;
- Figure 6: shows a plan view of a keyed element corresponding to the slot of figure 5a;
- Figure 7: shows a tool in accordance with an embodiment.

### Detailed description

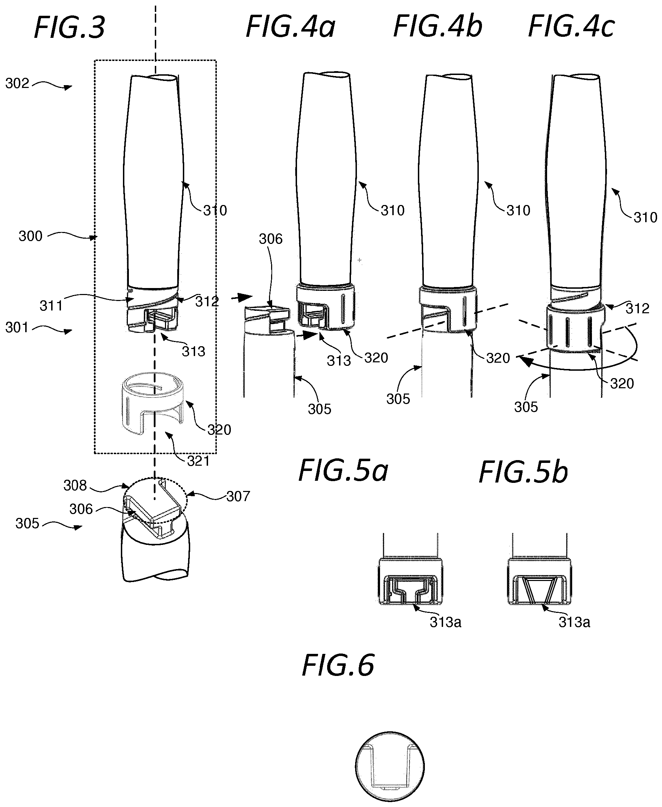

Figure 3 shows a handle for a modular tool in accordance with an embodiment. As shown, the handle 300 is provided with a body 310 having a distal end 301 and a proximal end 302. The distal end 301 provides a releasable coupling for a secondary element 305 of the modular tool. The releasable coupling comprises a cylindrical member 311 provided with a first external helical thread 312, and a lateral slot 313, the slot opening on one periphery of the distal end 301 of the body 310, the slot widening from the distal end 301 towards the proximal end 302. The releasable coupling further comprises an internally threaded ring 320, the threads of the ring 320 engaging the first external helical thread 312 of the cylindrical member 311, the threaded ring 320 being rotatable about the distal end 301 of thecylindrical member 311.

Where a plurality of helical threads are provided, one or more of these threads may have dimensions different to other threads, such that the ring can only engage the cylindrical member in the desired orientation, for example so as to ensure proper positioning of the optional detent with respect to the slot in the extended and retracted positions. For example, the helical thread has a first root to crest ratio, and the second helical thread has a second root to crest ratio, wherein the threads of the ring are defined so that the ring can only engage the corresponding respective helical thread, and thereby may only be mounted on the handle in one configuration. Similarly, the circumferential spacing of the threads may be varied to achieve the same effect.

The threads on the cylindrical member and/or the ring need not be continuous.

As shown, the ring is provided with an optional detent 321, in the distal circumferential edge thereof.

It will be appreciated that there is provided a secondary element having features in correspondence to the handle of the present invention, and in particular to the releasable coupling thereof. In particular, there is provided a secondary element for a modular tool, the secondary element having a distal end and a proximal end disposed on a longitudinal axis, the distal end of said secondary element being enclosed within a notional cylinder 307. The secondary element comprises a first keyed element 306 widening in a distal direction. A trailing edge 308 of the keyed element conforms to the cylinder 307, whereby said secondary element may be secured in a corresponding handle by inserting said first keyed element 306 with the trailing edge 308 outward into a corresponding slot defined in said handle, whereby a ring 320 of said handle corresponding to said cylinder may be slid into position so as to block said keyed element 306 in said handle.

According to the invention, the trailing edge 308 of the keyed element which conforms to the cylinder is furthermore partially threaded so as to provide a continuous thread with threads provided in a cylindrical member of the handle, and adapted to engage threads of the ring as described above.

Figures 4a, 4b and 4c show the handle of figure 3 in different configurations.

As shown, the threaded ring 320 is rotatable about the distal end 301 of the member 311 from a first, retracted position as shown in figures 4a and 4b, to a second, extended as shown in figure 4c.

Figure 4a shows the handle in a first, retracted position prior to the insertion of the secondary element 305. The secondary element may comprise a tool. For example, the tool may comprise a surgical instrument. More particularly for example, the tool may be for surgery of the orthopaedic surgery or bonetraumatology. Still more particularly for example tool may comprise a rasp or reamer or impactor. Alternatively, the secondary element may comprise an intermediate adaptor between the handle and a further component, where the further component may comprise a tool as discussed above or otherwise.

As shown, the secondary element 305 comprises a tongue or keyed element 306 which has a form complementary to the slot 313. As shown the ring 320 is in a retracted position in which the ring leaves the slot opening on one periphery of the distal end of the body unobstructed. By this means, the keyed element 306 of the secondary element 306 may be introduced into the slot 313 of the handle.

As shown, the optional detent 321 aligns with the slot in the retracted position.

Figure 4b shows the handle in a first, retracted position after insertion of the secondary element 305.

Since the slot 313 widens from the distal end 301 towards the proximal end 302, a correspondingly formed keyed element 306 may be slid into the slot laterally as shown in figure 4b, but once in position, will not be movable along the axis of the handle, i.e. towards or away from the distal end of the handle, but only back or forth along the axis of the slot.

Figure 4c shows the handle in a second, extended position after insertion of the secondary element 305. As shown in figure 4c, the ringhas been rotated about the cylindrical member 311, so that through the engagement of the threads of the ring in the threads 312 of the cylindrical member, this rotational movement has been translated into a linear movement towards the distal end of the handle, so that the ring obstructs the slot opening on one periphery of the distal end of the body.

As shown, this movement comprises a rotation of 180° clockwise about the axis of the handle when viewed down the length from the proximal to the distal end of the handle. The skilled person will appreciate that the pitch of the threads and the angle through which the ring is rotated will determine the distance along the cylindrical member that the ring 320 travels. On one hand it is desirable that the distance travelled should be as great as possible, to allow the use of a long keyed element 306, providing a strong and rigid connection between the handle and secondary element. On the other hand, in use it will be desirable that the user be required to turn the circle through as small an angle as possible, so that the manipulation may be performed with the lowest possible demand on the user's dexterity. The chosen compromise between these considerations is defined by the pitch of the respective threads of the ring and the cylindrical member. On this basis, the angular displacement of the ring between the extended position and the retracted position is preferably less than 361°, more preferably less than 271°, more preferably less than 181°. A multiple of 90° may be advantageous in terms of being more intuitive to the user. The angular displacement of the ring between the extended position and the retracted position is preferably more than 89°. The chosen compromise between these considerations is defined by the pitch of the respective threads of the ring and the cylindrical member.

Additional constraints may occur through a desire to limit the likelihood of the ring moving along the cylindrical member under its own weight, or in response to an accidental or incidental pressure, which will tend to favour a tighter pitch, although these considerations may also be managed by selecting materials, surfaces and/or tolerances so that the friction between elements reduces the risk of such unwanted movement. On this basis, the threads of the ring and cylindrical member may be configured in accordance with an embodiment such that the ring is turned through substantially one single revolution or less between the extended position and the retracted position. In accordance with a further embodiment the ring is turned through substantially one half revolution or less between the extended position and the retracted position.In accordance with a further embodiment the ring is turned through substantially one quarter revolution or less between the extended position and the retracted position. In accordance with a further embodiment the ring is turned through substantially one third revolution or more between the extended position and the retracted position. Meanwhile,the threads of the ring and cylindrical member may be configured in accordance with an embodiment such that the ring moves along the axis of the cylindrical member a distance substantially equal to 2cm or less between the extended position and the retracted position. In accordance with a further embodiment the ring moves along the axis of the cylindrical member a distance substantially equal to 1.5cm or less between the extended position and the retracted position.In accordance with a further embodiment the ring moves along the axis of the cylindrical member a distance substantially equal to 1cm or less between the extended position and the retracted position. The skilled person will appreciate that any combination of angle of rotation and linear displacement may be selected within these ranges, and indeed outside these ranges. It may be noted that the thread of the cylindrical member and ring of figures 4a, 4b and 4c comprises a double thread. A double thread may be advantageous in providing a firm engagement between the threads of the ring and the cylinder while permitting a short threaded section along the length of the ring, so as to minimise the dimensions of the ring. The skilled person will appreciate that a single thread may also be adequate in many implementations. The skilled person will appreciate that a three or even more threadsmay be appropriate in other implementations.

As shown in figures 3 and 4a, the slot 313 defines substantially a T cross section, and the keyed element 306 is shaped correspondingly. This shape achieves the objective of ensuring that the keyed element can only enter or exit the slot through the lateral opening of the cylindrical section, and that once the ring is in the extended position, no force in any direction on the secondary element with respect to the handle will separate the secondary element from the handle. The skilled person will recognise that the keyed element and corresponding slot may have any form complying with the general requirement that it widens from the distal end towards the proximal end. As such, it may form a wedge, dovetail or T section as described above. It may furthermore be circular, elliptical, rectangular, square, or any other form. The keyed element will generally constitute an extrusion of the chosen cross section from one side to the other. In certain embodiments, the keyed element may taper from one side to the other. Where this is the case the slot may taper from side to side correspondingly. Where this is the case, the slot may be defined as being deeper from side to side that the length from side to side of the corresponding keyed element. On this basis, the tapering walls of the keyed element will engage the sides of the keyed element before the end of the keyed element reached the lateral extremity of the slot. By this means, the slot will become progressively tighter as the keyed element is inserted, and a firm insertion without any play between the handle and secondary element may be achieved by pushing the keyed element fully into the slot.

Figure 4c shows the handle in a second, extended position after insertion of the secondary element 305.

As shown, the ring 320 has been rotated through 180° clockwise about the cylindrical member 311, and by the action of the engagement between the threads 312 on the cylindrical member 311 with those of the ring, the ring has progressed along the length of the cylindrical member so as to block the slot 313. It may further be noted that while in the retracted position as shown in figures 4a and 4b the optional detent 321 aligns with the slot, in the extended position of figure 4c the optional detent 321 no longer aligns with the slot. As such, the provision of the slot 321 means that the slot can be effectively blocked by a smaller linear movement of the ring.

As such, as shown the keyed element of the secondary element 305 is entirely trapped in the slot by the new position of the ring, as the result of a simple half turn (in the present example) of the ring, which may be effectuated by the user with a movement of the thumb, retaining the tool in the other fingers of the actuating hand, and leaving the other hand free. To further facilitate this action, the ring may be knurled, grooved, provided with a non-slip coating,provided with flats or otherwise treated to improve the users grip thereon. Where the ring is provided with flats, these may be even in number, and may further be dimensioned so that they may be engaged using a spanner of standard dimensions, for example as defined in ISO/TC 29/SC 10 and the like.

In certain embodiments, the shape or dimension of ring and cylindrical member, and in particular the thread on either or both of the ring or the cylindrical member may vary along their length, for example such that their engagement becomes progressively tighter towards the extended position, so that while it may move freely at certain portions of its travel, as it approaches the extended position it becomes somewhat resistant to movement. This approach may be advantageous in further reducing the risk of the ring being moved from the extended position inadvertently, which might otherwise lead to a loosening or even decoupling of the secondary element from the handle. Similarly, the engagement between the ring and cylindrical member may become progressively tighter towards the retracted position, so that while it may move freely at certain portions of its travel, as it approaches the retracted position it becomes somewhat resistant to movement. This approach may be advantageous in further reducing the risk of the ring being moved from the retracted position inadvertently, which might otherwise complicate the task of inserting the keyed element of the secondary element. The variations in shape or dimensions may include for example variations in the pitch, the minor diameter, the major diameter, or the root to crest ratio or any combination of these. Additionally or alternatively a similar effect may be achieved by a variation in the outer diameter of the cylindrical member and/or the inner diameter of the ring along the length thereof, for example so that as the ring moves towards the extended position, it becomes progressively tighter on the cylindrical member and/or so that as the ring moves towards the retracted position, it becomes progressively tighter on the cylindrical member.

As discussed above, the slot (and correspondingly the keyed element) widens from the distal end towards the proximal end. As discussed above, the slot, and correspondingly the keyed element may take many forms whilst satisfying this requirement. Figures 5a and 5b show two possible such implementations.

Figure 5a shows a sectional view of the slot in a first embodiment.

As shown in figure 5a, the slot 313a is generally T shaped in cross section. It may be noted that the shoulders defining the top of the narrow stem of the T shape are not entirely parallel with the flat top of the T shape. In other embodiments, these shoulders may be parallel with the top, or adopt any other angle as may be found to be expedient.

Figure 5b shows a sectional view of the slot in a second embodiment.

As shown in figure 5b, the slot 313b is generally in the form of a dovetail in cross section.

As shown in both figures 5a and 5b, the dimensions of the lateral slot vary radially (that is, from the edge towards the middle) so that the force required to slide a corresponding keyed element of the tool into the lateral slot increases as the corresponding keyed element of the tool progresses into the slot.

Figure 6 shows a plan view of a keyed element corresponding to the slot of figure 5a.

The keyed element of figure 6 corresponds to that of the secondary element 305 shown in figure 3. As shown, the slight tapering of the keyed element is visible.

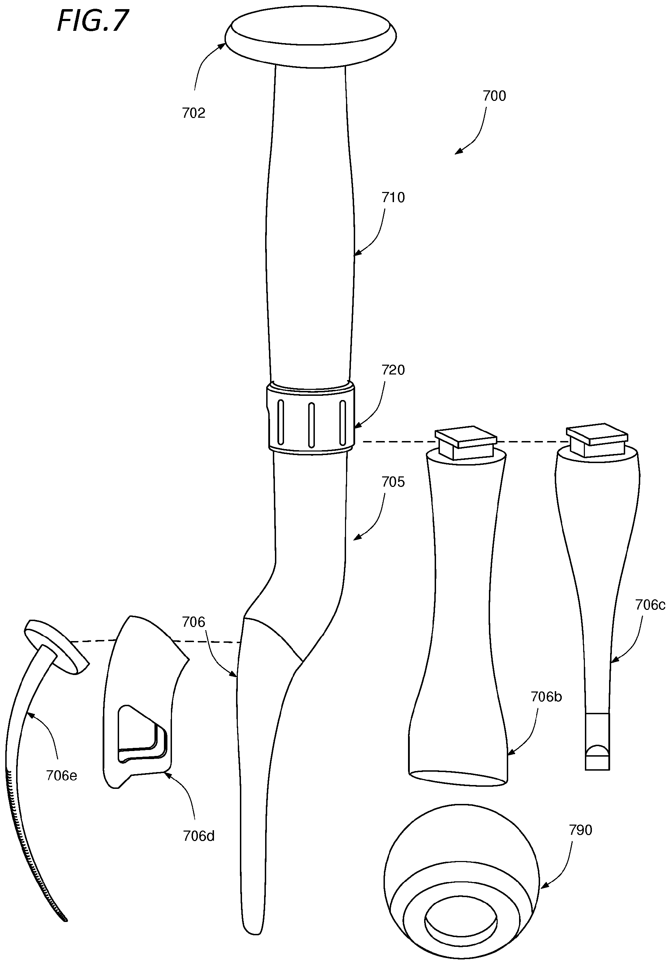

Figure 7 shows a tool in accordance with an embodiment.

As shown, there is provided a modular tool 700 comprising a handle 710 substantially as described above, and a secondary element 705 substantially as described above. The handle 710 comprises a releasable coupling substantially as described above, and in particular comprising a threaded ring 720, the threads of the ring engaging an external helical thread of the cylindrical member of the handle 700. The threaded ring 720 is rotatable about the distal end of the member between an extended position as shown in which the ring obstructs a slot opening on one periphery of the distal end of the body, and a retracted position in which the ring leaves the slot opening on one periphery of the distal end of the body unobstructed.

The handle may optionally be provided with an angle datum such as a radial line on the guard plate, or a radial lumen through which a bar may by inserted.

As shown, the handle further comprises an optional guard plate 702 at the proximal end thereof. Such a guard plate may serve to protect the hand of a user when gripping the handle 710 from blows struck against the proximal end thereof with a hammer, mallet or the like, for example where the tool 705 is a chisel, reamer or other such tool requiring a percussive application.

One field in which a handle as described may be appropriate is that of surgical instruments, such that the modular tool as a whole may compriseor constitutes a surgical instrument. More particularly, the modular tool may be for orthopaedic surgery or bone traumatology. More particularly, the modular tool may be is for surgery of the hip, shoulder or knee. More particularly, as shown, a working part 706, and thus the modular tool as a whole 700 comprises a rasp or reamer or impactor. It will be appreciated that in line with the many fields of application and associated tool types that may be envisaged, many different possible working parts 706 may be envisaged, for use with a single handle in accordance with embodiments as described above. Further examples of possible working parts, and resulting modular tools, include head impactor 706b (adapted to exert a force on an implant head 790), implant impactor 706c, which are examples of working parts that may be attached directly to the handle without a separate intervening secondary element 705, a curved rasp 706e, osteotome 706d which are further examples of working parts that may be attached directly to the handle by means of a separate intervening secondary element 705 and many other tools as will readily occur to the skilled person.

While the working part may incorporate the keyed part as described above, there may alternatively be provided a separate interface part as described above being releasably coupled to the handle as described above, and a working part being releasably coupled to the interface part, by a further releasable coupling means, which may comprise an additional ring mechanism as described above, a Hudson fitting as known in the state of the art, or any other convenient mechanism.

The handle of the present invention may be formed of any material. In particular, it may be formed of steel, aluminium, titanium or any other suitable metal or alloy. It may also be formed of a thermoplastic or other synthetic material. It may in particular be formed from a polyamide, for example a polyarylamide. The synthetic material may comprise additional components such as a filler, swelling agent and the like. It may still further be formed of a synthetic composite material, comprising a glass, carbon fibre, carbon nanoparticle or any other material exhibiting a high tensile strength, in a matrix of a synthetic material, such as any of those listed above. In certain embodiments, the handle of claim 13wherein the handle is composed of a glassfibre reinforced polyacrylamide, such as for example that marketed by the Solvay corporation under the trademark "Ixef 1022" ormore particularly "Ixef GS 1022".

The handle may be formed of different materials in different regions, including metal parts and synthetic parts. The handle may also comprise voids for the purpose of economy of material, reduced weight and so on.

It will be understood that the configurations and/or approaches described herein are exemplary in nature, and that these specific embodiments or examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

The subject matter of the present disclosure includes all novel and non-obvious combinations and sub-combinations of the various processes, systems and configurations, and other features, functions, acts, and/or properties disclosed herein, as well as any and all equivalents thereof.

Accordingly there is provided a handle for a modular tool in which the handle may be releasably coupled to a working part such as a rasp, reamer or impactor, or to a secondary element which may itself be connected to a working part. The coupling comprises a cylindrical member provided with one or more external helical thread, and a lateral slot opening on one periphery of the handle. A threaded ring has threads engaging the threads of the cylindrical member, and is rotatable about the cylindrical member between an extended position in which the ring obstructs the slot opening, and a retracted position in which the ring leaves the slot opening unobstructed. Accordingly, when the ring is in the retracted position a keyed element of the working part or secondary element may be slide from the side into the slot of the handle, and then once the ring is rotated to the extended position, the working part or secondary element is locked in place.

It will be understood that the configurations and/or approaches described herein are exemplary in nature, and that these specific embodiments or examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

The subject matter of the present invention is defined by the appended claims.

## Claims

1. A handle (300) for a modular tool, the handle comprising a body (310) having a distal end (301) and a proximal end (302), said distal end providing a releasable coupling for an element of said modular tool, said releasable coupling comprising:
a cylindrical member (311) provided with a first external helical thread (312), and a lateral slot (313), said slot opening on one periphery of said distal end of said body, said slot widening from said distal end towards said proximal end,
said releasable coupling further comprising a threaded ring (320), the threads of said
ring engaging said first external helical thread of said cylindrical member, said threaded ring being rotatable about said distal end of said member between an extended position in which said ring obstructs said slot opening on one periphery of said distal end of said body, and a retracted position in which said ring leaves said slot opening on one periphery of said distal end of said body unobstructed.

2. The handle of claim 1 wherein said lateral slot defines a T slot or dovetail.

3. The handle of claim 1 or 2 wherein the dimensions of said lateral slot vary radially so that the force required to slide a corresponding keyed element of said secondary element into said lateral slot increases as said corresponding keyed element of said tool progresses into said slot.

4. The handle of any preceding claim wherein the angular displacement of said threaded ring when rotated between said extended position and said retracted position is between 170 and 190 degrees.

5. The handle of any preceding claim wherein said threaded ring comprises an indentation (321) at the distal edge thereof, said indentation being positioned on the circumference of said ring such that when said ring is in said retracted position said indentation is aligned with said slot.

6. The handle of claim 5 wherein said cylindrical member is provided with a second external helical thread parallel said first external helical thread, and wherein the threads of said ring engage both said first external helical thread and said second external helical thread of said cylindrical member.

7. The handle of claim 6 wherein said first helical thread has a first root to crest ratio, and said second helical thread has a second root to crest ratio, wherein the threads of said ring are defined so that said ring can only engage the corresponding respective helical thread, and thereby may only be mounted on said handle in one configuration.

8. The handle of any preceding claim wherein the pitch, the minor diameter, the major diameter, or the root to crest ratio of said of said first external helical thread varies along the length of said first external helical thread so that the force required to rotate said ring increases as the ring is rotated about said distal end of said member from said retracted position to said extended position.

9. The handle of any preceding claim wherein said handle is composed of a synthetic material or a synthetic composite material.

10. The handle of claim 9 wherein said handle is composed of a glass fiber reinforced polyarylamide.

11. A secondary element (305) for a modular tool, said secondary element having a distal end and a proximal end disposed on a longitudinal axis, the distal end of said secondary element being enclosed within a cylinder (307), said secondary element comprising a first keyed element (306) widening in a distal direction, wherein a trailing edge (308) said keyed element conforms to said cylinder, whereby a working part (706) is configured to be secured in a corresponding handle (300) according to any of the preceding claims by inserting said first keyed element with said trailing edge outward into said corresponding slot (313) defined in said handle, whereby said ring (320) of said handle corresponding to said cylinder (307) is configured to be rotated into position so as to block said keyed element in said handle, wherein said trailing edge (308) of said keyed element conforms to the cylinder (307); **characterized in that** said keyed element is partially threaded so as to provide a continuous thread with threads provided in said cylindrical member (311) of said handle, and adapted to engage threads of said ring.

## Patentansprüche

1. Handgriff (300) für ein modulares Werkzeug, wobei der Handgriff einen Körper (310) mit einem distalen Ende (301) und einem proximalen Ende (302) umfasst, wobei das distale Ende eine lösbare Kopplung für ein Element des modularen Werkzeugs bereitstellt, wobei die lösbare Kopplung umfasst:
ein zylindrisches Element (311), versehen mit einem ersten Außenschraubengewinde (312), und
einen seitlichen Schlitz (313), wobei sich der Schlitz an einem Umfang des distalen Endes des Körpers öffnet, wobei sich der Schlitz von dem distalen Ende zu dem proximalen Ende erweitert,
wobei die lösbare Kopplung ferner einen Gewindering (320) umfasst, wobei die Gewinde des Rings mit dem ersten Außenschraubengewinde des zylindrischen Elements in Eingriff stehen, wobei der Gewindering um das distale Ende des Elements zwischen einer ausgefahrenen Position, in welcher der Ring die Schlitzöffnung an einem Umfang des distalen Endes des Körpers versperrt, und einer zurückgezogenen Position drehbar ist, in der der Ring die Schlitzöffnung an einem Umfang des distalen Endes des Körpers ungehindert lässt.

2. Handgriff nach Anspruch 1, wobei der seitliche Schlitz einen T-Schlitz oder einen Schwalbenschwanz definiert.

3. Handgriff nach Anspruch 1 oder 2, wobei die Abmessungen des seitlichen Schlitzes radial variieren, sodass die Kraft, die erforderlich ist, um ein entsprechendes Keilelement des sekundären Elements in den seitlichen Schlitz zu schieben, entsprechend dem Vordringen des entsprechenden Keilelements des Werkzeugs in den Schlitz zunimmt.

4. Handgriff nach einem der vorhergehenden Ansprüche, wobei die Winkelverschiebung des Gewinderings zwischen 170 und 190 Grad beträgt, wenn er zwischen der ausgefahrenen Position und der zurückgezogenen Position gedreht wird.

5. Handgriff nach einem der vorhergehenden Ansprüche, wobei der Gewindering eine Einkerbung (321) an seiner distalen Kante umfasst, wobei die Einkerbung am Umfang des Rings so positioniert ist, dass, wenn sich der Ring in der zurückgezogenen Position befindet, die Einkerbung mit dem Schlitz ausgerichtet ist.

6. Handgriff nach Anspruch 5, wobei das zylindrische Element mit einem zweiten Außenschraubengewinde versehen ist, welches parallel zu dem ersten Außenschraubengewinde ist, und wobei die Gewinde des Rings sowohl in dem ersten Außenschraubengewinde als auch in dem zweiten Außenschraubengewinde des zylindrischen Elements eingreifen.

7. Handgriff nach Anspruch 6, wobei das erste Schraubengewinde ein erstes Wurzel-Scheitel-Verhältnis aufweist und das zweite Schraubengewinde ein zweites Wurzel-Scheitel-Verhältnis aufweist, wobei die Gewinde des Rings so definiert sind, dass der Ring nur in das entsprechende jeweilige Schraubengewinde eingreifen kann und dadurch auf dem Handgriff nur in einer einzigen Konfiguration darauf montiert werden kann.

8. Handgriff nach einem der vorhergehenden Ansprüche, wobei die Teilung, der kleinere Durchmesser, der Hauptdurchmesser oder das Wurzel-Scheitel-Verhältnis des ersten Außenschraubengewindes entlang der Länge des ersten Außenschraubengewindes variiert, so dass die zum Drehen des Rings erforderliche Kraft zunimmt, wenn der Ring um das distale Ende des Elements von der eingefahrenen Position in die ausgefahrene Position gedreht wird.

9. Handgriff nach einem der vorhergehenden Ansprüche, wobei der Handgriff aus einem synthetischen Material oder einem synthetischen Verbundmaterial besteht.

10. Handgriff nach Anspruch 9, wobei der Handgriff aus einem glasfaserverstärkten Polyarylamid besteht.

11. Sekundäres Element (305) für ein modulares Werkzeug, wobei das Sekundärelement ein distales Ende und ein proximales Ende aufweist, welche auf einer Längsachse angeordnet sind, wobei das distale Ende des sekundären Elements in einem Zylinder (307) eingeschlossen ist, wobei das sekundäre Element ein erstes Keilelement (306) umfasst, das sich distal erweitert, wobei eine Hinterkante (308) des Keilelements dem Zylinder anpasst, wodurch ein Arbeitsteil (706) konfiguriert ist, um in einem entsprechenden Handgriff (300) nach einem der vorhergehenden Ansprüche befestigt zu werden, indem das erste Keilelement mit der Hinterkante nach außen in den entsprechenden Schlitz (313) eingeführt wird, der in dem Handgriff definiert ist, wodurch der Ring (320) des Handgriffs entsprechend dem Zylinder (307) konfiguriert ist, um in Position gedreht zu werden, um das Keilelement in dem Handgriff zu blockieren, wobei die Hinterkante (308) des Keilelements dem Zylinder (307) anpasst; **dadurch gekennzeichnet, dass** das Keilelement teilweise mit einem Gewinde versehen ist, um ein durchgehendes Gewinde bereitzustellen, wobei Gewinde in dem zylindrischen Element (311) des Handgriffs vorgesehen und angepasst sind, um mit Gewinden des Rings in Eingriff zu kommen.

## Revendications

1. Poignée (300) pour un outil modulaire, la poignée comprenant un corps (310) ayant une extrémité distale (301) et une extrémité proximale (302), ladite extrémité distale fournissant un couplage séparable pour un élément dudit outil modulaire, ledit couplage séparable comprenant :
un élément cylindrique (311) doté d'un premier filetage hélicoïdal externe (312), et
une fente latérale (313), ladite fente s'ouvrant sur une périphérie de ladite extrémité distale dudit corps, ladite fente s'élargissant à partir de ladite extrémité distale vers ladite extrémité proximale,
ledit couplage séparable comprenant en outre une bague filetée (320), les filetages de ladite bague se mettant en prise avec ledit premier filetage hélicoïdal externe dudit élément cylindrique, ladite bague filetée étant apte à tourner autour de ladite extrémité distale dudit élément entre une position déployée dans laquelle ladite bague obstrue ladite ouverture de fente sur une périphérie de ladite extrémité distale dudit corps, et une position rétractée dans laquelle ladite bague laisse ladite ouverture de fente sur une périphérie de ladite extrémité distale dudit corps non obstrué.

2. Poignée selon la revendication 1, dans laquelle ladite fente latérale définit une fente en T ou une queue d'aronde.

3. Poignée selon la revendication 1 ou 2, dans laquelle les dimensions de ladite fente latérale varient radialement de sorte que la force requise pour faire coulisser un élément claveté correspondant dudit élément secondaire dans ladite fente latérale augmente à mesure que ledit élément claveté correspondant dudit outil progresse dans ladite fente.

4. Poignée selon l'une quelconque des revendications précédentes, dans laquelle le déplacement angulaire de ladite bague filetée lorsqu'elle tourne entre ladite position déployée et ladite position rétractée se situe entre 170 et 190 degrés.

5. Poignée selon l'une quelconque des revendications précédentes, dans laquelle ladite bague filetée comprend une échancrure (321) au niveau du bord distal de celle-ci, ladite échancrure étant positionnée sur la circonférence de ladite bague de telle sorte que lorsque ladite bague est dans ladite position rétractée ladite échancrure est alignée avec ladite fente.

6. Poignée selon la revendication 5, dans laquelle ledit élément cylindrique est doté d'un second filetage hélicoïdal externe parallèle audit premier filetage hélicoïdal externe, et dans laquelle les filetages de ladite bague se mettent en prise avec à la fois ledit premier filetage hélicoïdal externe et ledit second filetage hélicoïdal externe dudit élément cylindrique.

7. Poignée selon la revendication 6, dans laquelle ledit premier filetage hélicoïdal a un premier rapport entre la base et le sommet, et ledit second filetage hélicoïdal a un second rapport entre la base et le sommet, dans laquelle les filetages de ladite bague sont définis de sorte que ladite bague peut uniquement se mettre en prise avec le filetage hélicoïdal respectif correspondant, et de ce fait peut uniquement être montée sur ladite poignée dans une configuration unique.

8. Poignée selon l'une quelconque des revendications précédentes, dans laquelle l'écartement, le diamètre mineur, le diamètre majeur ou le rapport entre la base et le sommet dudit premier filetage hélicoïdal externe varie le long de la longueur dudit premier filetage hélicoïdal externe de sorte que la force requise pour faire tourner ladite bague augmente à mesure que la bague tourne autour de ladite extrémité distale dudit élément à partir de ladite position rétractée vers ladite position déployée.

9. Poignée selon l'une quelconque des revendications précédentes, dans laquelle ladite poignée est composée d'un matériau synthétique ou d'un matériau composite synthétique.

10. Poignée selon la revendication 9, dans laquelle ladite poignée est composée d'un polyarylamide renforcé par des fibres de verre.

11. Elément secondaire (305) pour un outil modulaire, ledit élément secondaire ayant une extrémité distale et une extrémité proximale disposées sur un axe longitudinal, l'extrémité distale dudit élément secondaire étant comprise au sein d'un cylindre (307), ledit élément secondaire comprenant un premier élément claveté (306) s'élargissant dans une direction distale, dans lequel un bord de fuite (308) dudit élément claveté se conforme audit cylindre, moyennant quoi une partie opérationnelle (706) est configurée pour être fixée dans une poignée (300) correspondante selon l'une quelconque des revendications précédentes en insérant ledit premier élément claveté avec ledit bord de fuite vers l'extérieur dans ladite fente (313) correspondante définie dans ladite poignée, moyennant quoi ladite bague (320) de ladite poignée correspondant audit cylindre (307) est configurée pour être tournée dans une position de manière à bloquer ledit élément claveté dans ladite poignée, dans lequel ledit bord de fuite (308) dudit élément claveté se conforme au cylindre (307) ; **caractérisé en ce que** ledit élément claveté est partiellement fileté de manière à fournir un filetage continu avec des filetages fournis dans ledit élément cylindrique (311) de ladite poignée, et adaptés pour se mettre en prise avec des filetages de ladite bague.
